# EUROPEAN PATENT SPECIFICATION

(11) **EP 4 427 684 B1**
(45) Date of publication and mention of the grant of the patent: **07.01.2026**
(21) Application number: 21834819.1
(22) Date of filing: 03.11.2021
(51) Int. Cl.: A61B 17/15

(54) **PERSONALIZED SURGICAL GUIDE FOR VALGUS-DEROTATIONAL OR THREE-DIMENSIONAL TIBIAL OSTEOTOMY**
PERSONALISIERTE CHIRURGISCHE FÜHRUNG FÜR VALGUS-DEROTATIONS- ODER DREIDIMENSIONALE TIBIALE OSTEOTOMIE
GUIDE CHIRURGICAL PERSONNALISÉ POUR OSTÉOTOMIE TIBIALE DE VALGISATION OU TRIDIMENSIONNELLE

(43) Date of publication of application: 11.09.2024
(73) Proprietor: Digital Anatomics, S.L., 28919 Leganés (ES)
(72) Inventor: REYERO HUERGA, Alejandro, 24004 León (León) (ES); IGLESIA CABANEIRO, Vicenta, 24193 Villaquilambre (León) (ES)
(74) Representative: San Martin Alarcia, Esther
(86) International application number: PCT/ES2021/070791
(87) International publication number: WO 2023/079190

(56) References cited:
- EP-A1- 3 763 304
- CN-A- 112 168 278
- US-A1- 2018 049 749
- US-A1- 2020 352 582

## Description

### OBJECT OF THE INVENTION

The present invention belongs to the field of medicine, and more particularly to the field of orthopaedic surgery and traumatology.

The object of the present invention is a personalized surgical guide designed for carrying out a valgus--derotational or three-dimensional tibial osteotomy

### PRIOR ART

Tibial osteotomy is a surgical procedure for correcting certain defects in connection to the orientation of the tibial bone. Essentially, tibial osteotomy consists of making a cut near the upper end of the tibia and, thereafter, rotating the main body of the tibia under the cut with respect to one or several planes for improving its alignment. Thereto, there exist currently a number of techniques which can be classified depending on the position and orientation of the cut, as well as on the type of rotation of the tibia.

### Open-wedge tibial osteotomy

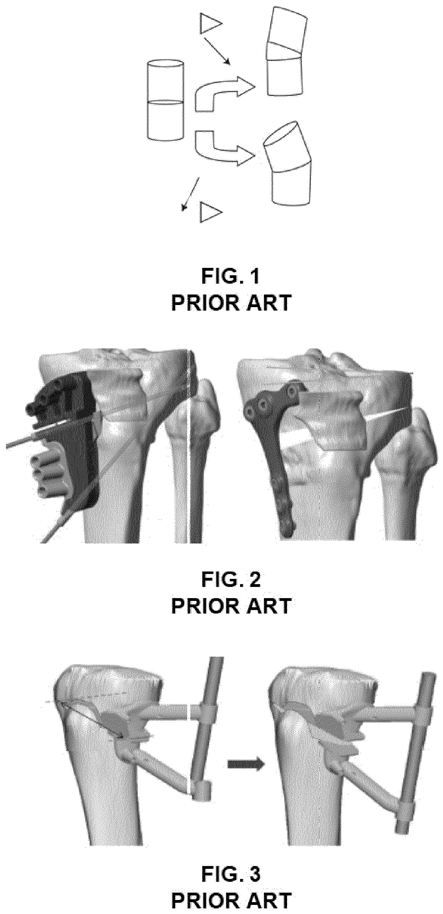

This surgical technique is also known as open-wedge HTO (High Tibial Osteotomy). This procedure corrects the valgus or varus of the patient by carrying out a partial cut at the high portion of the tibia and by inserting a wedge in the cut (or, alternatively, by extracting a wedge of bone material). As an example, the scheme of Fig. 1 shows this kind of operation where an essentially horizontally oriented cut is made (taking the natural position of the leg of the patient with the patient standing as a reference) and a wedge is introduced therein (or a wedge of bone material is extracted therefrom) for achieving a correct alignment of a longitudinal axis of the tibia that, initially, was inclined in a coronal plane of the patient.

To improve the precision of these operations, the use of a patient-personalized guide designed for guiding the cutting tool for ensuring a suitable depth, position and orientation of the cut is known. Document EP3804636A1 discloses a guide of this type developed by Newclip International. Article *"*Posteromedial opening-wedge tibial osteotomy for metaphyseal varus and abnormal posterior slope correction in failed anterior cruciate ligament reconstructions using a custom cutting guide", Boris Corin *et al,* discloses the use of this guide. Fig. 2, belonging to said article, shows how said guide (in black) is mainly formed by two 3D-printed parts mating each other. The posterior surface of the guides is a negative of the shape of the bone of the patient exactly at the place where they must be placed, thereby ensuring a correct positioning of the guide as a whole. Once positioned, the two parts are fixed to the bone by means of respective sets of Kirschner wires. Between the two parts, a groove making up a guide for carrying out the cut is defined. An additional Kirschner wire inserted from below with an upwards inclination provides a stop for the cutting tool. After extracting the guide, the above-mentioned Kirschner wires aid in positioning and orienting the cut in the bone and in limiting its depth. After cutting the bone, the cut is opened by rotating the main body of the bone until a predetermined planned position where the initial misalignment is corrected is arrived at, and then finally the new position of the bone is fixed by means of a fixation plate. Although not shown in the figures, a wedge made of natural or artificial bone material is introduced in the cut.

A second type of personalized guide similar to the one disclosed above is also known that, in addition to ensuring a correct position and depth of the cut, provides control in connection to the opening angle of the bone. Document EP3403600A1 discloses a guide of this type developed by A Plus Biotechnology Company Limited. Article *"*Clinical experience using a 3D-printed patient-specific instrument for medial opening wedge high tibial osteotomy", by Jesse Chieh-Szu Yang *et al,* discloses the use of this guide. Fig. 3, included in said article, shows how the guide has a similar structure to the above disclosure: two complementary parts that are fixed to the bone and between which there is a groove for guiding the cut. However, in addition to these elements the guide has two arms protruding from said parts and having respective orifices at their ends. The guide is 3D-printed, such that these orifices are only aligned when the cut is opened a predetermined angle calculated in advance, and then a rod is introduced for fixing the two bone portions in said position. This method allows for ensuring not only a correctly positioned cut having a suitable depth, but also a rotation of the bone according to the planning.

In short, the surgical technique disclosed heretofore allows for correcting the position of the tibia in a single plane by opening the bone in a particular direction by introducing a wedge in the cut (or by closing the bone by the extraction of a wedge of bone from the cut). In particular, the plane of the cut is essentially perpendicular to a coronal plane of the patient, such that rotation of the bone takes place within said coronal plane of the patient (as expected for correcting the valgus or the varus). The guides disclosed above are consequently designed for being placed against a lateral surface of the tibia for aiding in carrying out a cut having the correct position, orientation and depth and, in certain cases, for aiding in opening the cut according to the correct angle.

### Conventional derotational tibial osteotomy

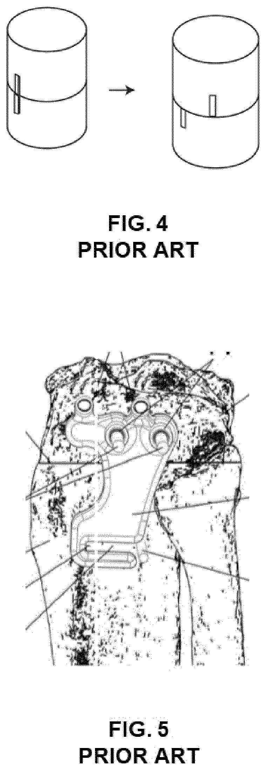

Conventional derotational tibial osteotomy mainly consists of carrying out a complete cut of the tibia in a plane essentially perpendicular to the axis of the tibia and, thereafter, rotating the tibia essentially around its own axis. That is, the cut is contained in an essentially horizontal plane when the patient is standing. Mechanically, it is a relatively simple operation where only the orientation of the tibia of the patient is corrected, as easily seen in the scheme of Fig. 4.

The main difficulty of this surgical procedure is ensuring that the bone is rotated exactly the angle calculated during the planning step. Thereto, Santxarizmendi Grupo de Investigación has developed the guide disclosed in document EP3763304A1. This document discloses a personalized 3D-printed guide for aiding the surgeon in rotating the tibia the predetermined angle. The device is mainly formed by a plate intended to be fixed to the bone portion located above the cut, and it comprises a groove at a lower area along which, when the main body of the bone located under the cut is rotated, a Kirschner wire moves. When the Kirschner wire reaches the end of the groove, that is, its closed end, that means that the bone has turned the planned angle. Note that this device does not provide help for carrying out the cut itself, which is supposed to have been made in advance with the help of a specific guide also developed by the same company, this specific guide further having means for fixing in a particular position of the bone of the patient the Kirschner wire used as a reference for the subsequent rotation.

### Valgus-derotational or three-dimensional tibial osteotomy

Thirdly, valgo-derotational tibial osteotomy surgeries are known. The purpose of a valgus-derotational tibial osteotomy, also known as three-dimensional tibial osteotomy or ICATME osteotomy (since it was developed by the Instituto Catalán de Traumatologia y Medicina Deportiva), is to correct in a single procedure both the angular deviation of the tibia, normally varus, and the torsion of the tibia. In these operations, the orientation of the cut is no longer perpendicular to the coronal plane of the patient (or essentially horizontal when the patient is standing), but is has an inclination with respect to both the coronal plane and the sagittal plane. As shown in Fig. 4, the cut is complete, and the tibia is rotated essentially along its own axis a predetermined angle. Consequently, deviations in two different planes are corrected in a single surgical act. Article "Cómo realizar una osteotomía tridimensional de rodilla. Estudio preliminar del cálculo matemático", by J. Gash i Blasi et al discloses this technique in detail.

As disclosed above, this technique is advantageous in comparison with the abovementioned techniques in that misalignments in two planes can be corrected with a single surgical procedure. However, this technique is difficult mainly due to two factors. First, unlike as in the osteotomy techniques disclosed above, it is very complicated to ensure a correct position of the cutting plane, since it now has an *"arbitrary"* orientation with respect to the main planes of the patient, that is, there are now no useful references such as the perpendicularity to the coronal plane of the prior art techniques. Second, once the cut is made, rotating the bone exactly the angle calculated during the planning step is is also very complicated. In view of these complications, not may surgeons nowadays use this technique.

Chinese patent application CN112168278 discloses a personalized surgical guide (1) for three dimensional tibia osteotomy, that is personalized for patient and 3D-printed according to a previous step of surgical planning, comprising a first part (11) having a posterior face (18) whose shape is the negative of an area of a first portion of the bone of the patient, said first part (11) comprising orifices (141) for fixation by means of fixation wires to said first portion of bone; and a second part (12) having a posterior face (18) whose shape is the negative of an area of the second portion of bone of the patient adjacent the said area of the first portion of bone, the second part (12) comprising orifices (141) for fixation by means of fixation wires to said second portion of bone; where the first part (11) and the second part (12) are configured to be coupled along a cutting groove (10) serving as a guide for cutting the bone for separating the first portion of bone from the second portion of bone, wherein, when the surgical guide (1) is fixed to the bone in a position where the posterior face (18) of the first part (11) fits in the first portion of bone and the posterior face (18) of the second part (12) fits on the second portion of bone, the first part (11) and the second part (12) being coupled one to the other, the cutting groove (10) is contained in a plane that is inclined with respect to both the sagittal plane of the patient.

There is currently no surgical guide designed specifically for solving this problem, that is, for facilitating the precise correction of a deviation of the tibia in both the coronal plane and the sagittal plane in a single procedure.

### DESCRIPTION OF THE INVENTION

The present invention is defined in the claims and discloses a new personalized surgical guide designed for aiding in performing a valgus-derotational or three-dimensional tibial osteotomy.

The personalized surgical guide of the present invention mainly has two differentiating features with respect to the heretofore known guides: first, when fixed in position on the bone, the cutting groove and a number of upper Kirschner wires aligned adjacent the cutting groove of this new surgical guide are positioned in an plane inclined with respect to the main planes of the patient (coronal and sagittal); second, the new surgical guide has means for signalling the moment when the bone has been rotated the correct planned angle. These means may be two fixation wires suitably positioned thanks to specific orifices of the surgical guide such that, even after the surgical guide is withdrawn from the bone, said fixation wires provide a visual indication aiding the surgeon in knowing how much to rotate the bone for precisely arriving at the value calculated during the planning step.

The guide disclosed in document CN112168278 is the closest prior art known to the inventors, and therefore the preamble of the first claim is based on said document.

A number of terms used in the present specification are now disclosed.

Anterior/posterior: The posterior side or face of an element is the side or face that, in the natural use position of said element, is oriented towards the bone of the patient. Similarly, the anterior side or face of an element is the side or face that, in the natural use position of said element, is oriented in a direction opposite the bone of the patient. The anterior/posterior direction goes from the anterior side towards the posterior side of an element essentially in perpendicular to the bone surface when the element is fixed in position.

Upper/lower: The upper side or face of an element is the side or face that, in the natural use position of said element and assuming the patient is standing, is situated at a higher height with respect to the rest of said element. Similarly, the lower side or face of an element is the side or face that, in the natural use position of said element and assuming the patient is standing, is situated at a lower height with respect to the rest of the element. That is, terms *"upper"* and *"lower"* have their conventional meaning assuming that the element in question is placed in position on the bone of the patient and that the longitudinal axis of the bone of the patient is essentially coincident with the vertical direction.

Lateral: The lateral direction is perpendicular to the anterior/posterior direction and the superior/inferior direction such that, when the guide is positioned on the bone of the patient, it corresponds to the directions commonly known as lateral right and lateral left taking the patient him/herself as the reference. That is, when the guide is positioned, they are essentially horizontal directions in perpendicular to the anterior/posterior direction

Sagittal/coronal planes: These terms have the same meaning they conventionally have in the field of medicine. The sagittal plane is the plane of symmetry of the human body through the centre of the chest. The coronal plane is a plane perpendicular to the sagittal plane and dividing the person in a back portion and a forward portion. When the person is standing, both the sagittal plane and the coronal plane are vertical.

Bone longitudinal axis: It is the main axis extending along the bone. In particular, for the tibia, this axis is essentially vertical when the patient is standing.

Fixation wire: Wire, needle, pin or any similar element conventionally used for the fixation of guides and other auxiliary devices to the bone of the patient. Kirschner wires are just an example of fixation wire, while others may be used.

The surgical guide of the present invention is configured for carrying out a valgus-derotational or three-dimensional tibial osteotomy. The surgical guide is personalized for the anatomy of the patient and 3D-printed according to a previous surgical planning process known in the art. In short, said planning process comprises: first, analyzing medical images of the anatomy of the patient, for example imagens obtained by NMR (Nuclear Magnetic Resonance), CAT (Computerized Axial Tomography) and others; and then, determining the mechanical parameters defining the surgical operation to be performed, mainly the position and orientation of the cutting plane and the rotation angle of the bone. Once the planning is finished, the surgical guide is designed according to the anatomy of the patient and to the mechanical parameters obtained during the planning process. Finally, the surgical guide is 3D-printed.

The surgical guide of the invention mainly comprises two parts that can be coupled one to the other, known as first part and second part.

The first part has a posterior face whose shape is the negative of an area of a first portion of the bone of the patient, where said first part comprises orifices for being fixed by means of fixation wires to said first portion of bone. The second part has a posterior face whose shape is the negative of an area of a second portion of the bone of the patient adjacent said zone of the first portion of bone, where said second part also comprises orifices for being fixed by means of fixation wires to said second portion of bone. Further, the first part and the second part can be coupled one to the other along a cutting groove serving as a guide for cutting the bone for separating the first portion of bone from the second portion of bone.

The elements disclosed above are known from the prior art, for example, from the surgical guide disclosed in document EP3804636A1, and therefore they make up the preamble of the independent claim of the present application. However, the guide disclosed in the present document differs from the known guide mainly in two characteristics: orientation of the plane of the cutting groove; and signalling of the rotation angle of the bone. In the following, each of said characteristics is disclosed in more detail.

### Orientation of the plane of the cutting groove

The plane of the cutting groove for cutting the bone in the surgical guide of the present invention has a different orientation in comparison with the surgical guides known heretofore. In known surgical guides, the plane of the cutting groove, when the guide is correctly positioned on the bone of the patient, is essentially horizontal (that is, perpendicular to the sagittal and coronal planes of the patient). However, in the guide of the invention, the plane of the cutting groove when the guide is in the natural use position is not horizontal, but it is inclined with respect to both the horizontal plane and the main planes of the patient, as was to be expected for carrying out a valgus-derotational or three-dimensional tibial osteotomy.

In other words, in the present invention, when the surgical guide is fixed to the bone in a position such that the posterior face of the first part fits on the first portion of bone and the posterior face of the second part fits on the second portion of bone, with the first part and the second part coupled one to the other, the cutting groove is contained in a plane inclined with respect to the sagittal plane and the coronal plane of the patient.

This feature is advantageous in that the surgical guide can be used for carrying out valgus-derotational or three-dimensional tibial osteotomies, since the surgeon is made aware of the position and orientation where the cut must be performed, thus improving the precision of the surgical procedure.

### Signalling the rotation angle of the bone

The first part and the second part of the surgical guide of the present invention further comprise means configured for signalling a moment when the second portion of bone has rotated a predetermined rotation angle with respect to the first portion of bone.

This feature is advantageous in that the surgeon is provided with a rotation angle indication preventing imprecisions during the bone rotation process, thus cooperating in improving the precision of the surgical procedure.

In principle, the means for signalling the rotation angle can be implemented in different manners through particular means respectively located at the first part and at the second part. For example, said particular means may be guides or positioning elements intended to leave marks at certain positions of the bone of the patient, the position of said marks being designed to provide the surgeon with a reference in connection with the rotation angle the main body, normally the second portion of bone, must rotate to achieve the rotation value calculated during planification.

However, according to a preferred embodiment of the invention, the means for signalling the moment when the second portion of bone has rotated a predetermined rotation angle with respect to the first portion of bone comprise the following elements:
a) A first reference orifice of the firs part configured for insertion of a first reference wire into the first portion of bone.
b) A second reference orifice of the second part configured for insertion of a second reference wire into the second portion of bone.

Thus, once the first part and the second part of the surgical guide are separated from the bone after the cut contained in said inclined plane of the cutting groove is performed, a particular relative position of the second reference wire with respect to the first reference wire indicates that the second portion of bone has rotated the predetermined rotation angle with respect to the first portion of bone.

This configuration is advantageous in that elements conventionally employed in these types of surgeries, such as fixation wires, are used for providing the surgeon with an indication in connection to the rotation angle of the bone.

In principle, the relative position between the first and the second reference wires indicating that the predetermined rotation angle is achieved could be anyone. For example, relationships defined by straight lines in parallel or in perpendicular to the main planes of the patient (sagittal, coronal, or transversal) or to the main axis of the bone may be used, since these directions and planes are easily recognizable during the surgery. More particularly, according to a preferred embodiment of the invention, the relative position of the second reference wire with respect to the first reference wire indicating that the second portion of bone is rotated the predetermined angle of rotation with respect to the first portion of bone consists on the alignment, in a direction in parallel to the longitudinal axis of the bone, of the insertion point into the bone of the second reference wire with respect to the insertion point into the bone of the first reference wire.

This configuration is advantageous in that it is easy for the surgeon to determine when the insertion point of the first reference wire and the insertion point of the second reference wire are aligned according to the axis of the bone. The axis of the bone is oriented in a direction contained in planes parallel to the coronal plane and to the sagittal plane such that, when the patient is standing, it is essentially vertical.

In principle, the first reference orifice indicating the position where the first reference wire is fixed may be located at any position of the first part. However, the lowest the distance between the first reference wire and the second reference wire, the more precisely the surgeon can determine an alignment between them. For this reason, according to a particularly preferred embodiment of the invention, the first reference orifice of the first part is located in a position adjacent a lower edge of said first part making up an upper face of the cutting groove. That is, the first reference wire will be fixed very close to the upper edge of the second part, and therefore as close as possible to the point where the second reference orifice will be located. Similarly, although the second reference orifice may be located at any position of the second part, preferably it is located at a position adjacent an upper edge of said second part forming a lower face of the cutting groove.

As mentioned above, this configuration is advantageous in that the closest the reference wires are positioned, the more precisely the surgeon can determine that they are aligned.

According to another particularly preferred embodiment of the invention, an anterior face of the second part comprises a mark adjacent an upper edge of the second part making up a lower face of the cutting groove, said mark indicating the position where the first reference wire must be located when the second portion of bone has rotated the predetermined rotation angle with respect to the first portion of bone.

This configuration is advantageous because, in case the second reference orifice is not located near the upper edge of the second part, the second reference wire is relatively far from the first reference wire, and therefore the precision of the determination of the rotation angle would decrease. Since it is situated closer to the first reference wire, the mark allows the surgeon to more precisely checking the alignment.

According to still another preferred embodiment of the invention, the mark is a straight line connecting a point adjacent said upper edge of the second part where the first reference wire must be situated when the second portion of bone has rotated the predetermined rotation angle with the second reference orifice of the second part. That is, the mark is a visual indication of the imaginary line parallel to the axis of the bone (that is, vertical considering the patient is standing) that must connect the first reference wire and the second reference wire when the desired rotation angle is achieved.

This configuration is advantageous in that it makes easier for the surgeon to recognize the moment when both wires are aligned by providing a straight line that he/she must only extend upwards, either imaginarily or with the aid of auxiliary tools, to check whether it hits the first reference wire.

Additionally, the surgical guide of the invention may comprise a series of additional elements intended to ensure a good adjustment between the first part and the second part. Now, some of these elements are briefly disclosed.

According to another preferred embodiment of the invention, the upper edge of the second part comprises a projection configured to abut against the lower edge of the first part for separating said edges for making up the groove when both parts are coupled. The projection may be shaped as an elevated area with respect to the upper edge of the second part situated at a lateral side of said upper edge. Therefore, the projection, that is situated at a lateral side of the upper edge of the second part, makes up a separator between said upper edge of the second part and the lower edge of the first part to ensure that between them the groove serving as a cutting guide is formed. The height of the projection is therefore essentially coincident with the width of the groove.

This configuration is advantageous in that the desired width of the groove between the first and the second parts when both are coupled one to the other is ensured.

According to still another preferred embodiment of the invention, the upper edge of the first part further comprises a boss protruding from the projection, and the lower edge of the first part comprises a cavity open towards the posterior surface, the boss and the cavity being complementary. Therefore, when the second part couples with the first part, the boss enters the cavity and slides along said cavity until an anterior face of the second part abuts against a posterior face of an auxiliary body of the first part.

This configuration is advantageous in that coupling between the first part and the second part is ensured, and also in that a correct positioning of a part with respect to the other part is facilitated.

According to a particularly preferred embodiment of the invention, a posterior edge of the projection is separated from the posterior face of the second part a predetermined distance such that, when the second part is fixed onto the area of the first portion of bone with the upper edge positioned at the cutting plane, the second portion of bone abuts against said posterior edge of the projection when it has rotated the predetermined rotation angle with respect to the first portion of bone. That is, the projection is made such that is posterior edge, which is that located closest to the bone when the second part is coupled thereto, is separated from the posterior surface of the second part a distance that is precalculated during surgery planification. Thereby, once the bone is cut, with the second part placed in position, and with the first part taken away, the plane of the upper edge of the second part is coincident with the cutting plane, and the second portion of bone, when rotating, slides over said upper edge of the second part until bumping with the projection, which impedes any further rotation. This is an indication that the desired rotation angle is achieved.

This configuration is advantageous in that another way to check that the second portion of bone has rotated the desired predetermined angle is provided. It can be used as the only means to signal que moment when the second portion of bone has rotated a predetermined rotation angle or, alternatively, it can be used in combination with the abovementioned reference orifices such that one of the two means provides a confirmation that the bone has effectively been rotated the desired angle.

According to another preferred embodiment of the invention, the anterior face of the second part comprises guide pins that are complementary with recesses at the posterior face of the auxiliar body of the first part such that, when the second part is coupled to the first part, the guide pins fit into the recesses.

This configuration is advantageous in that the coupling between the first part and the second part is made more firm, sliding of one part with respect to the other being prevented.

### BRIEF DESCRIPTION OF THE DRAWINGS

Fig. 1 schematically shows the cut made in an open-wedge high tibial osteotomy.
Fig. 2 shows an example of a known guide device for carrying out an open-wedge high tibial osteotomy.
Fig. 3 shows another example of a known guide device for carrying out an open-wedge high tibial osteotomy.
Fig. 4 schematically shows the cut made in a conventional derotational tibial osteotomy.
Fig. 5 shows an example of a known guide device for carrying out a conventional derotational tibial osteotomy.
Fig. 6 schematically shows the cut made in a valgus-derotational or three-dimensional tibial osteotomy.
Fig. 7 shows a perspective view of an example of surgical guide for carrying out a valgus-derotational or three-dimensional tibial osteotomy according to the present invention.
Fig. 8a and 8b show respective views of a first part of the exemplary surgical guide according to the present invention.
Figs. 9a and 9b show respective views of a second part of the exemplary surgical guide according to the present invention.
Figs. 10a-10h sequentially show several steps of the method for using the surgical guide of the present invention.
Fig. 11 shows a detailed view of the alternative method for checking the angle rotated by the bone.

### PREFERRED EMBODIMENT OF THE INVENTION

An example of a surgical guide (1) according to the present invention particularly applied to a tibial osteotomy is now disclosed.

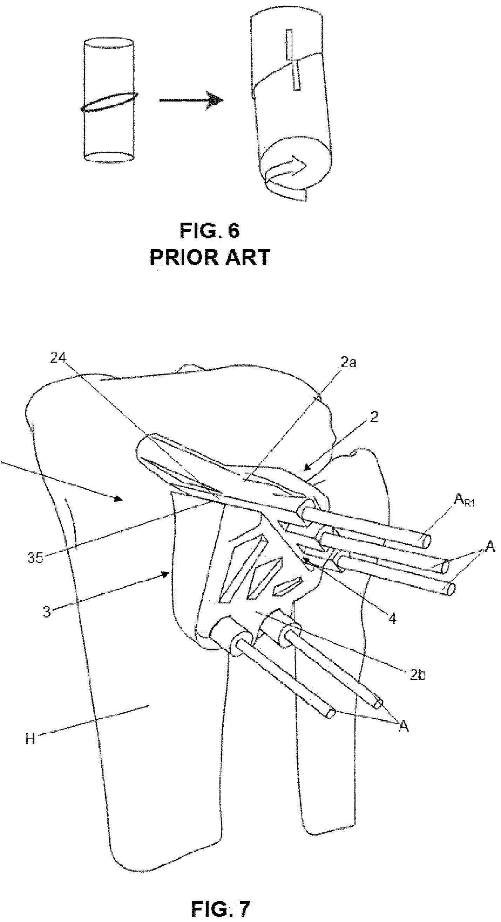

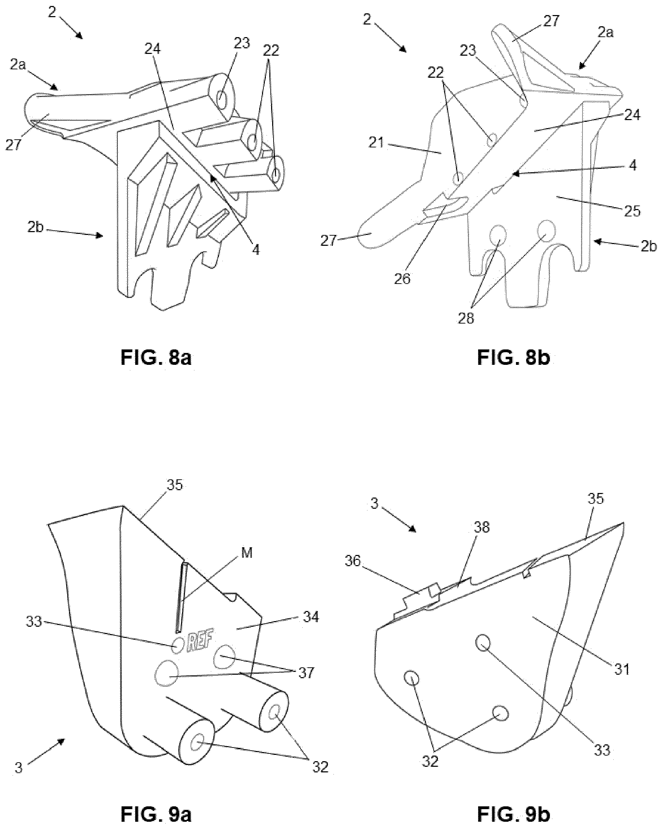

Fig. 7 shows the surgical guide (1) according to the present invention when fixed to the bone at its natural use position, while Figs. 8a and 8b and Figs. 9a and 9b show respective views of the two parts (2, 3) making up said surgical guide (1).

As shown, the surgical guide (1) is positioned on an area of the bone (H) of the patient, in particular the tibia of the patient, located near its upper end (that is, the end of the tibia where the connection with the femur of the patient is located). The surgical guide (1) is formed by a first part (2) and a second part (3) which, in use, are coupled one to the other. Both parts are personalized for each patient by 3D-printing from medical images acquired during an earlier surgical planning stage.

In particular, the first part (2) has a posterior face (21) having a shape that corresponds to the negative of the shape of the bone (H) of the patient at an area of the first portion of bone (H) it is intended to be placed against. The first portion of bone (H) is, in this example, the upper end of the tibia, such that the posterior face (21) of the first part has a shape that corresponds to the negative of an area of the upper end of the tibia.

The second part (3) has a posterior face (31) having a shape that corresponds to the negative of the shape of the bone (H) of the patient in an area of the second portion of bone (H) it is intended to be placed against. In this example, the second portion of bone (H) is the main body of the tibia once the osteotomy is carried out. The posterior face (31) of the second part (3) therefore has a shape corresponding to the negative of an area of said main body of the tibia situated near its upper end. Naturally, the area of the second portion of bone (H) where the second part (3) is placed is adjacent the area of the first portion of bone (H) where the first part (3) is placed.

The first part (2) and the second part (3) have means for coupling one to the other when they are fixed to the bone (H) of the patient such that between them a groove (4) is formed, the groove being contained in a plane which, when they are both fixed at their position onto the bone (H), is inclined with respect to both the sagittal plane of the patient and the coronal plane of the patient. This groove (4) serves for marking the exact position of the cut to be made during the surgery. The groove (4), which can have a width of about 1 mm to 3 mm, is defined by a lower edge (24) of the first part (2) that, when both parts are coupled, is adjacent and parallel to an upper edge (35) of the second part (3). Both edges (24, 35) are essentially flat and fit at an lateral end of the groove (4).

In particular, a boss (36) provided at a lateral side of the upper edge (35) of the second part (3) fits into a cavity (26) correspondingly provided at a lateral side of the lower edge (24) of the first part (2). The boss (36) is provided at a projection (38) of the upper edge (35) of the second part (3). In this example, said projection (38) is an essentially rectangular elevated area in parallel to the plane of the upper edge (35). The projection (38) does not extend for the whole length of the edge (35) in the anterior-posterior direction, but it is separated from a posterior corner where the edge (35) meets the posterior surface (31) of the second part. The main function of this projection (38) is ensuring a separation between the lower edge (24) of the first part (2) and the upper edge (35) of the second part making up the groove (4). Indeed, when both parts are coupled one to the other, the surface of the projection (38) is the contact point where the lower edge (24) of the first part (2) abuts against the upper edge (35) of the second part (3). Naturally, the height of the projection (38) corresponds with the width of the groove (4) mentioned earlier (for example, 1-3 mm). The projection (38) can also have an additional function for signalling the desired rotation angle during the surgical procedure, as disclosed further down in the present document.

Going back to the complementary elements for coupling the first part (2) and the second part (3), the cavity (26) of the first part (2) is open at an anterior side thereof, that is, at the side that is coincident with the anterior face (21) of said first part (2). Therefore, starting from a position where the first and second parts (2, 3) are fixed to the bone by means of fixation wires (A) and coupled one to the other, it is possible to extract the first part (2) pulling it outwards to make the boss (36) slide along the cavity (26) until exiting through the open anterior side. Conversely, to assemble the guide (1) of the invention, the second part (3) is made to slide with respect to the first part (2) such that the boss (36) enters the cavity (26). The area where the connection between the cavity (26) of the first part (2) and the boss (36) of the second part (3) takes place is a closed end of the groove (4), that is open at its opposite end.

The first part (2) is additionally formed by two distinct bodies: a main body (2a) and an auxiliary body (2b). The main body (2a) and the auxiliary body (2b) are connected at the plane of the cutting groove (4). The posterior face of the main body (2a) is the posterior face (21) of the part (2) whose form is the negative of the bone (H) of the patient, and the lower edge of the main body (2a) is the lower edge (24) of the first part (2) to which the upper edge (35) of the second part (3) is coupled. In turn, the auxiliary body (2b) projects from an anterior area of said lower edge (24) of the main body (2a) of the first part (2). Since between the main body )2a) and the auxiliary body (2b) the cutting groove (4) is located, both bodies (2a, 2b) are connected only at an area located at a lateral end of body, specifically at a lateral side adjacent the position of the cavity (26).

Further, the posterior face (25) of the auxiliary body (2b) is configured to abut against an anterior face (34) of the second part (3) when both are coupled one to the other in the manner disclosed in the previous paragraph. To prevent the parts from sliding with respect to the other, the anterior face (34) of the second part (3) comprises additionally a pair of guide pins (37) configured to fit into respective recesses (28) provided at the posterior face (25) of the auxiliary body (2b). Thus, as shown in Fig. 7, when both parts (2, 3) are fixed to the bone (H) in their natural use position, the second part (3) is located under the second portion (2b) of the first part (2).

In addition to the elements mentioned until this point, the first part (2) additionally has two wings (27) emerging laterally and backwardly from the main body (2a). A posterior face of both wings (27) is part of the posterior face (21) of the first part (2), and it therefore has a personalized shape for abutting against the bone (H) of the patient. These wings (27) further improve the precision in connection with positioning the second part (2) against the bone (H) of the patient by laterally extending the contact surface between the guide (1) and the bone (H).

Both parts (2, 3) additionally comprise a number of orifices intended for receiving fixation wires, e.g. Kirschner wires. In particular, the first part (2) has three orifices: a first reference orifice (23) for the fixation of a first reference wire (A_{R1}) and two fixation orifices (22) for the fixation of two fixation wires (A). These orifices pass through the first part (2) in an anterior-posterior direction, from an anterior face to the posterior face (21). The function of the three wires (A, A_{R1}) is fixing the first part (2) of the guide (1) to the bone (H) of the patient at the predetermined position mentioned above, although the first reference wire (A_{R1}) further has the function of cooperating for signalling the rotation angle of the second portion of bone (H), as disclosed in details further down in the present document.

Although not an indispensable condition, the fixation orifices (22) and the reference orifice (23) are all adjacent the cutting groove (4) and they are aligned in parallel to the plane of said cutting groove (4), thus aiding to guide the cutting tool during the bone cutting process.

Similarly, the second part (2) has three orifices: a second reference orifice (33) for the insertion of a second reference wire (A_{R2}) and two fixation orifices (32) for the insertion of fixation wires (A). These orifices pass through the second part (3) in the anterior-posterior direction, from the anterior face (34) to the posterior face (31). The function of these wires (A, A_{R2}) is to fix the second guide part (3) to the bone (H) of the patient at the above disclosed predetermined position, although the second reference wire (A_{R2}) further has the function of cooperating with the first reference wire (A_{R1}) for signalling the rotation angle in the manner disclosed further down in the present document. The two fixation orifices (32) are situated near the lower side of the second part (3), while the second reference orifice (33) is, in this example, situated at a central zone of the second part (3).

The second part (3) also has a mark (M) located at the surface of the anterior face (34) connecting the second reference orifice (33) with the upper edge (35) at the point where the first reference wire (A_{R1}) should be when the second portion of bone (H) has been rotated the desired angle. In the natural use position of the guide (1), that is, when the second part (3) is fixed to the bone (H) of the patient at the zone mentioned before, the mark (M) has a vertical direction, that is, parallel to the longitudinal axis (E_{L}) of the bone (H).

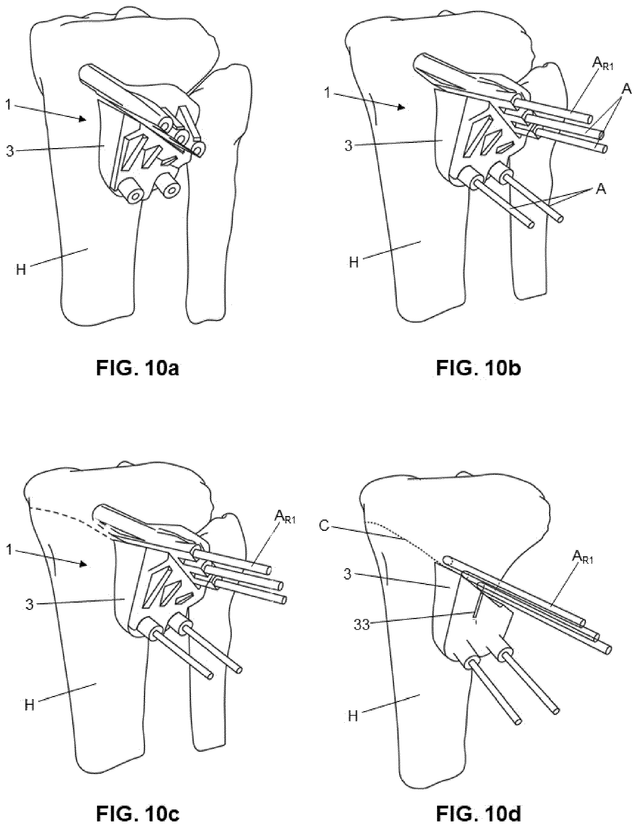

Figs. 10a-10h show different steps of the process for using the surgical guide (1) of the present invention. First, as shown in Fig. 10a, the two parts (2, 3) of the surgical guide (1) are placed in their respective positions on the bone (H) of the patient. The two parts (2, 3) are coupled one to the other by inserting the boss (36) of the upper edge (35) of the second part (3) into the cavity (26) of the lower edge (24) of the first part (2). The posterior face of the surgical guide (1) is thereby formed by the posterior face (21) of the first part (2) and the posterior face (31) of the second part (3), which together make up a surface whose shape is complementary with the shape of the bone (H) of the patient at the area where the cut is going take place. The posterior face (25) of the second body (2b) of the first part (2) abuts against the anterior face (34) of the second part (3). The cutting groove (4) is situated between the lower edge (24) of the first part (2) and the upper edge (35) of the second part (3) (and partly also between the first body (2a) and the second body (2b) of the first part (2)).

Then, the surgical guide (1) formed by these two parts (2, 3) thus coupled is precisely placed on the bone (H) at said position and the fixation wires (A, A_{R1}, A_{R2}) are inserted. In particular, two fixation wires (A) are inserted through the fixation orifices (22) of the first part (2) and the first reference wire (A_{R1}) is inserted through the first reference orifice (23) of the first part (2). In turn, two fixation wires (A) are inserted through the two fixation orifices (32) of the second part (3), since the auxiliary body (2b) of the first part (2) abuts on the anterior face (34) of the second part (3) in such a manner that those orifices (32) are not blocked. However, in this example the auxiliary body (2b) of the first part (2) covers the reference orifice (33) of the second part (3), and therefore the second reference wire (A_{R2}) is not inserted yet. The result of this step, as shown in Fig. 10b, is that the surgical guide (1) as a whole is already fixed to the bone (H) exactly at the desired position. In this position, the guide groove (4) is situated in a plane having the position and orientation that were predetermined during the surgical planning step. This plane, as mentioned before, and in correspondence to a valgus-derotational or three-dimensional osteotomy operation, is inclined both with respect to the coronal plane and with respect to the sagittal plane of the patient. Naturally, that necessarily means that it is also inclined with respect to the longitudinal axis (E_{L}) of the bone, that is, it is not perpendicular thereto.

Once the surgical guide (1) is fixed to the bone (H) at the desired position, the position where the cut is going to be performed is marked with the aid of the cutting groove (4), as shown in Fig. 10c.

Then, the first part (2) of the surgical guide (1) is withdrawn from the bone (H). Thereto, the first part (2) is pulled from such that the cavity (26) of the first part (2) slides along the boss (36) of the second part (3) and the wires (A, A_{R1}) slide along the respective orifices (22, 23). At the end of this step, which is shown in Fig. 10d, the anterior face (34) of the second part (3) is totally visible, and therefore both the second reference orifice (33) and the mark (M) are also visible.

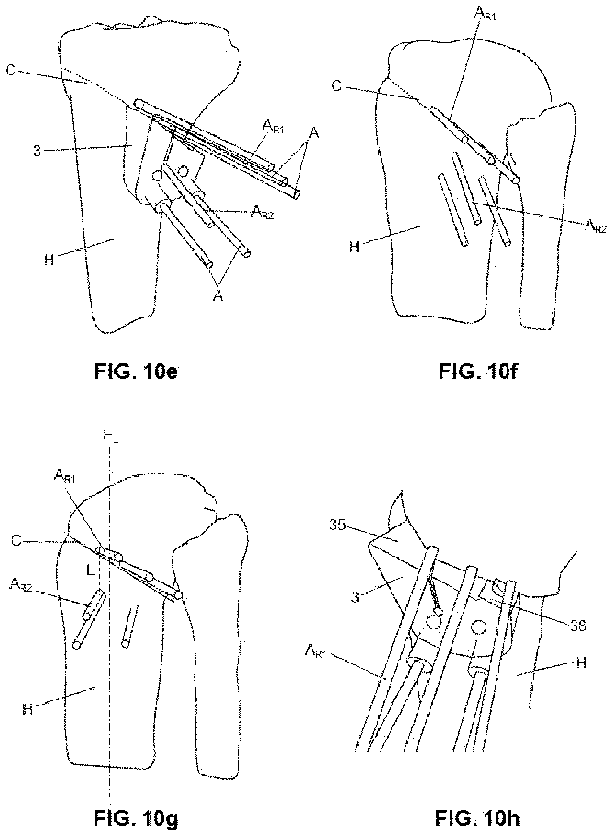

As shown in Fig. 10e, the second reference wire (A_{R2}) is then fixed through the second reference orifice (33).

Once the second reference wire (A_{R2}) is fixed, the second part (3) is extracted by pulling therefrom, such that the wires (A, A_{R2}) slide along the corresponding orifices (23, 33). At the end of this step, as shown in Fig. 10f, the bone (H) of the patient is naked except for the wires (A, A_{R1}, A_{R2}) previously inserted throughout the process. A complete cut of the bone (H) by means of a suitable cutting tool is then carried out. The wires (A, A_{R1}) inserted into the first portion of bone, that is, those that were inserted through the fixation orifices (22) and the first reference orifice (23) of the first part (2), make up a plane parallel to the cutting plane marked on the bone (H), thereby cooperating for guiding the cutting tool.

Once the bone (H) has been cut completely, the second portion of the bone (H), that is, the portion of bone (H) situated under the cut (C) and making up the main portion thereof, is rotated. By rotating the second portion of bone (H), the relative position of the wires (A, A_{R1}) fixed to the first portion of bone (H) by means of the first part (2) with respect to the wires (A, A_{R2}) to the second portion of bone (H) by means of the second part (3) is modified. In the present invention, the positions of the first reference orifice (23) of the first part (2) and the second reference orifice (33) of the second part (3) were precisely calculated during the surgical planning, such that the moment when the first reference wire (A_{R1}) is vertically aligned with respect to the second reference wire (A_{R2}) signals the moment when the planned rotation angle is achieved. This is represented in Fig. 10g, where a discontinuous line (L) in parallel to the longitudinal axis (E_{L}) of the bone (H) clearly signals that the insertion point of the first reference wire (A_{R1}) is aligned with the insertion point of the second reference wire (A_{R2}).

Lastly, as shown in Fig. 10h, the second part (3) can be inserted again to ensure that the first reference wire (A_{R1}) and the second reference wire (A_{R2}) are correctly aligned. Thereto, use is made of the mark (M) in the anterior face (34) of the second part (3) connecting the second reference orifice (33) with the position of the upper edge (35) of the second part (3) where the first reference wire (A_{R1}) must be situated. That is, when the second part (3) is placed again in its position, mark (M) is an essentially vertical line. It is then easy to see that the first reference wire (A_{R1}) is situated just at the upper end of said mark (M), and therefore that the second portion of bone (H) has rotated the desired angle.

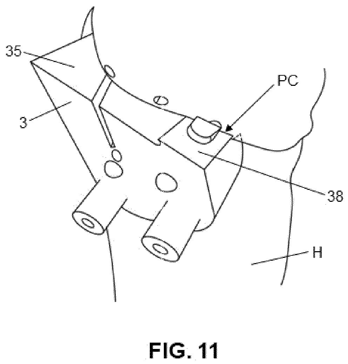

An alternative way to check that the second portion of bone (H) has been rotated the correct angle consists of checking that said second portion of bone (H) contacts the protrusion (38) of the upper edge (35) of the second part (3). That is, the second part (3) is inserted again in the manner disclosed in the previous paragraph, the upper edge (35) of the second part (3) being then in a position that is coincident with the plane of the cut (C). Since, as disclosed above, the posterior edge of the protrusion (38) is separated from the posterior surface (31) of the second part (3), before rotating the second portion of bone (H) a distance separates said bone (H) from said posterior edge of the protrusion (38). It is then possible to design the protrusion (38) such that its posterior edge is situated in a position where the second portion of bone (H) abuts just when it has rotated the desired angle. Fig. 11 graphically shows this contact point (PC).

In any case, once the second portion of bone (H) is rotated the desired angle and the relevant verifications have been made, the two portions of bone (H) are fixed one to the other by means of suitable elements commonly known in the field.

## Claims

1. Personalized surgical guide for valgus-derotational or three-dimensional tibia osteotomy, that is personalized for a patient and 3D-printed according to a previous step of surgical planning, comprising:
a first part (2) having a posterior face (21) whose shape is the negative of an area of a first portion of the bone (H) of the patient, said first part (2) comprising orifices (22) for fixation by means of fixation wires (A) to said first portion of bone (H); and
a second part (3) having a posterior face (31) whose shape is the negative of an area of a second portion of the bone (H) of the patient adjacent the said area of the first portion of bone (H), the second part (3) comprising orifices (32) for fixation by means of fixation wires (A) to said second portion of bone (H);
where the first part (2) and the second part (3) are configured to be coupled along a cutting groove (4) serving as a guide for cutting the bone (H) for separating the first portion of bone (H) from the second portion of bone (H), **characterized in that**
when the surgical guide (1) is fixed to the bone (H) in a position where the posterior face (21) of the first part (2) fits on the first portion of bone (H) and the posterior face (31) of the second part (3) fits on the second portion of bone (H), the first part (2) and the second part (3) being coupled one to the other, the cutting groove (4) is contained in a plane that is inclined with respect to both the sagittal plane and the coronal plane of the patient;
and **in that** the first part (2) and the second part (3) comprise means configured to signal a position where the second portion of bone (H) has rotated a predetermined rotation angle with respect to the first portion of bone (H) around an axis that is perpendicular to said inclined plane containing the cutting groove (4).

2. Surgical guide (1) according to claim 1, where the means for signalling the moment when the second portion of bone (H) has rotated a predetermined rotation angle with respect to the first portion of bone (H) comprise:
- a first reference orifice (23) of the first part (2) configured for the insertion therethrough of a first reference wire (A_{R1}) into the first portion of bone (H); and
- a second reference orifice (33) of the second part (3) configured for the insertion therethrough of a second reference wire (A_{R2}) into the second portion of bone (H),
such that, once the first part (2) and the second part (3) of the surgical guide (1) are separated from the bone (H) after the cut (C) contained in said inclined plane of the cutting groove (4) is performed, a particular relative position of the second reference wire (A_{R2}) with respect to the first reference wire (A_{R1}) indicates that the second portion of bone (H) has rotated the predetermined rotation angle with respect to the first portion of bone (H).

3. Surgical guide (1) according to claim 2, where the relative position of the second reference wire (A_{R2}) with respect to the first reference wire (A_{R1}) indicating that the second portion of bone (H) has rotated the predetermined rotation angle with respect to the first portion of bone (H) consists of the alignment, along a direction in parallel to the longitudinal axis (E_{L}) of the bone (H), of the insertion point in the bone (H) of the second reference wire (A_{R2}) with respect to the insertion point in the bone (H) of the first reference wire (A_{R1}).

4. Surgical guide (1) according to any of claims 2-3, where the first reference orifice (23) of the first part (2) is situated in a position adjacent a lower edge (24) of the first part (2) making up an upper face of the cutting groove (4).

5. Surgical guide (1) according to any of claims 2-4, where the second reference orifice (33) of the second part (3) is situated at a position adjacent an upper edge (35) of said second part (3) making up a lower face of the cutting groove (4).

6. Surgical guide (1) according to any one of claims 2-5, where an anterior face (34) of the second part (3) comprises a mark (M) adjacent an upper edge (35) of said second part (3) making up a lower face of the cutting groove (4), such that said mark (M) indicates the position where the first reference wire (A_{R1}) must be when the second portion of bone (H) has rotated the predetermined rotation angle with respect to the first portion of bone (H).

7. Surgical guide (1) according to claim 6, where said mark (M) is a straight line connecting a point adjacent said upper edge (35) of the second part (3) where the first reference wire (A_{R1}) must be situated when the second portion of bone (H) has been rotated the predetermined rotation angle with the second reference orifice (33) of the second part (3).

8. Surgical guide (1) according to any of the previous claims, where the upper edge (35) of the second part (3) comprises a projection (38) configured for abutting against the lower edge (24) of the first part (2) for separating said edges (24, 35) when both parts (2, 3) are coupled for making up the groove (4).

9. Surgical guide (1) according to claim 8, where the upper edge (35) of the first part (3) further comprises a boss (36) emerging from the projection (38) and the lower edge (24) of the first part (2) comprises a cavity (26) open towards the posterior surface (21), where the boss (36) and the cavity (26) are complementary such that, when the second part (3) is coupled to the first part (2), the boss (36) enters the cavity (26) and slides along said cavity (26) until the anterior face (34) of the second part (3) abuts against a posterior face (25) of an auxiliary body (2b) of the first part (2).

10. Surgical guide (1) according to any of claims 8-9, where a posterior edge of the projection (38) is separated from the posterior face (31) of the second part (3) a predetermined distance such that, when the second part (3) is fixed on the area of the first portion of bone (H) with the upper edge (35) situated in the plane of the cut (C), the second portion of bone (H) hits said posterior edge of the projection (38) when it has been rotated the predetermined rotation angle with respect with the first portion of bone (H).

11. Surgical guide (1) according to any of the previous claims, where the anterior face (34) of the second part (3) comprises guide pins (37) which are complementary with recesses (28) in the posterior face (25) of the auxiliary body (2b) of the first part (2) such that, when the second part (3) is coupled to the second part (2), the guide pins (37) fit into the recesses (28).

## Patentansprüche

1. Personalisierte chirurgische Schablone für eine Valgus-Derotations- oder dreidimensionale Tibiaosteotomie, die für einen Patienten personalisiert und gemäß einem vorherigen Schritt der Operationsplanung 3D-gedruckt ist, umfassend:
einem ersten Teil (2) mit einer hinteren Fläche (21), deren Form das Negativ eines Bereichs eines ersten Abschnitts des Knochens (H) des Patienten ist, wobei der erste Teil (2) Öffnungen (22) zur Befestigung mittels Befestigungsdrähten (A) an dem ersten Abschnitt des Knochens (H) umfasst; und
einem zweiten Teil (3) mit einer Rückseite (31), deren Form das Negativ eines Bereichs eines zweiten Abschnitts des Knochens (H) des Patienten ist, der an den genannten Bereich des ersten Abschnitts des Knochens (H) angrenzt, wobei der zweite Teil (3) Öffnungen (32) zur Befestigung mittels Befestigungsdrähten (A) an dem genannten zweiten Abschnitt des Knochens (H) umfasst;
wobei der erste Teil (2) und der zweite Teil (3) so konfiguriert sind, dass sie entlang einer Schneidnut (4) gekoppelt werden können, die als Führung zum Schneiden des Knochens (H) dient, um den ersten Teil des Knochens (H) vom zweiten Teil des Knochens (H) zu trennen, **dadurch gekennzeichnet, dass**
wenn die chirurgische Führung (1) an dem Knochen (H) in einer Position befestigt ist, in der die hintere Fläche (21) des ersten Teils (2) auf den ersten Teil des Knochens (H) und die hintere Fläche (31) des zweiten Teils (3) auf den zweiten Teil des Knochens (H) passt, wobei der erste Teil (2) und der zweite Teil (3) miteinander verbunden sind, die Schneidnut (4) in einer Ebene enthalten ist, die sowohl gegenüber der Sagittalebene als auch gegenüber der Koronalebene des Patienten geneigt ist;
und dass der erste Teil (2) und der zweite Teil (3) Mittel umfassen, die so konfiguriert sind, dass sie eine Position signalisieren, an der sich der zweite Teil des Knochens (H) um einen vorbestimmten Drehwinkel in Bezug auf den ersten Teil des Knochens (H) um eine Achse gedreht hat, die senkrecht zu der geneigten Ebene steht, die die Schneidnut (4) enthält.

2. Chirurgische Führung (1) nach Anspruch 1, wobei die Mittel zum Signalisieren des Moments, in dem sich der zweite Teil des Knochens (H) um einen vorbestimmten Drehwinkel in Bezug auf den ersten Teil des Knochens (H) gedreht hat, Folgendes umfassen:
- eine erste Referenzöffnung (23) des ersten Teils (2), die zum Einführen eines ersten Referenzdrahtes (A_{R1}) in den ersten Teil des Knochens (H) konfiguriert ist; und
- eine zweite Referenzöffnung (33) des zweiten Teils (3), die so konfiguriert ist, dass ein zweiter Referenzdraht (A_{R2}) durch sie hindurch in den zweiten Teil des Knochens (H) eingeführt werden kann,
sodass, sobald der erste Teil (2) und der zweite Teil (3) der chirurgischen Führung (1) nach dem Durchführen des in der geneigten Ebene der Schneidnut (4) enthaltenen Schnitts (C) vom Knochen (H) getrennt sind, eine bestimmte relative Position des zweiten Referenzdrahtes (A_{R2}) in Bezug auf den ersten Referenzdraht (A_{R1}) anzeigt, dass sich der zweite Knochen teil (H) um den vorbestimmten Drehwinkel in Bezug auf den ersten Teil des Knochens (H) gedreht hat.

3. Chirurgische Führung (1) nach Anspruch 2, wobei die relative Position des zweiten Referenzdrahtes (A_{R2}) in Bezug auf den ersten Referenzdraht (A_{R1}), die angibt, dass sich der zweite Abschnitt des Knochens (H) um den vorbestimmten Drehwinkel in Bezug auf den ersten Abschnitt des Knochens (H) gedreht hat, aus der Ausrichtung entlang einer Richtung parallel zur Längsachse (E_{L}) des Knochens (H) des Einführpunkts im Knochen (H) des zweiten Referenzdrahts (A_{R2}) in Bezug auf den Einführpunkt im Knochen (H) des ersten Referenzdrahts (A_{R1}) besteht.

4. Chirurgische Führung (1) nach einem der Ansprüche 2-3, wobei die erste Referenzöffnung (23) des ersten Teils (2) an einer Position neben einer Unterkante (24) des ersten Teils (2) angeordnet ist, die eine Oberseite der Schneidnut (4) bildet.

5. Chirurgische Führung (1) nach einem der Ansprüche 2 bis 4, wobei die zweite Referenzöffnung (33) des zweiten Teils (3) an einer Position neben einer Oberkante (35) des zweiten Teils (3) angeordnet ist, die eine Unterseite der Schneidnut (4) bildet.

6. Chirurgische Führung (1) nach einem der Ansprüche 2 bis 5, wobei eine Vorderfläche (34) des zweiten Teils (3) eine Markierung (M) benachbart zu einer Oberkante (35) des zweiten Teils (3) aufweist, die eine Unterseite der Schneidnut (4) bildet, so dass die Markierung (M) die Position angibt, an der sich der erste Referenzdraht (A_{R1}) sein muss, wenn der zweite Teil des Knochens (H) den vorbestimmten Drehwinkel in Bezug auf den ersten Teil des Knochens (H) gedreht hat.

7. Chirurgische Führung (1) nach Anspruch 6, wobei die Markierung (M) eine gerade Linie ist, die einen Punkt neben der oberen Kante (35) des zweiten Teils (3), an dem sich der erste Referenzdraht (A_{R1}) befinden muss, wenn der zweite Teil des Knochens (H) um den vorbestimmten Drehwinkel gedreht wurde, mit der zweiten Referenzöffnung (33) des zweiten Teils (3) verbindet.

8. Chirurgische Führung (1) nach einem der vorhergehenden Ansprüche, wobei die Oberkante (35) des zweiten Teils (3) einen Vorsprung (38) aufweist, der so ausgebildet ist, dass er an der Unterkante (24) des ersten Teils (2) anliegt, um die Kanten (24, 35) voneinander zu trennen, wenn beide Teile (2, 3) miteinander verbunden sind, um die Nut (4) zu bilden.

9. Chirurgische Führung (1) nach Anspruch 8, wobei die Oberkante (35) des ersten Teils (3) ferner einen Vorsprung (36) aufweist, der aus dem Vorsprung (38) herausragt, und die Unterkante (24) des ersten Teils (2) eine zur hinteren Fläche (21) hin offene Aussparung (26) aufweist, wobei der Vorsprung (36) und die Aussparung (26) komplementär zueinander sind, so dass, wenn der zweite Teil (3) mit dem ersten Teil (2) verbunden wird, der Vorsprung (36) in die Aussparung (26) eintritt und entlang dieser Aussparung (26) gleitet, bis die vordere Fläche (34) des zweiten Teils (3) an einer hinteren Fläche (25) eines Hilfskörpers (2b) des ersten Teils (2) anliegt.

10. Chirurgische Führung (1) nach einem der Ansprüche 8-9, wobei eine hintere Kante des Vorsprungs (38) von der hinteren Fläche (31) des zweiten Teils (3) um einen vorbestimmten Abstand getrennt ist, so dass, wenn der zweite Teil (3) auf dem Bereich des ersten Teil des Knochenss (H) mit der oberen Kante (35) in der Ebene des Schnitts (C) fixiert ist, der zweite Teil des Knochens (H) auf die hintere Kante des Vorsprungs (38) trifft, wenn er um den vorbestimmten Drehwinkel in Bezug auf den ersten Teil des Knochens (H) gedreht wurde.

11. Chirurgische Führung (1) nach einem der vorhergehenden Ansprüche, wobei die vordere Fläche (34) des zweiten Teils (3) Führungsstifte (37) umfasst, die komplementär zu Aussparungen (28) in der hinteren Fläche (25) des Hilfskörpers (2b) des ersten Teils (2) sind, so dass, wenn der zweite Teil (3) mit dem zweiten Teil (2) verbunden ist, die Führungsstifte (37) in die Aussparungen (28) passen.

## Revendications

1. Guide chirurgical personnalisé pour ostéotomie tibiale valgus-dérotationnelle ou tridimensionnelle, personnalisé pour un patient et imprimé en 3D selon une étape préalable de planification chirurgicale, comprenant :
une première partie (2) ayant une face postérieure (21) dont la forme est le négatif d'une zone d'une première partie de l'os (H) du patient, ladite première partie (2) comprenant des orifices (22) pour la fixation au moyen de fils de fixation (A) à ladite première partie de l'os (H) ; et
une deuxième partie (3) ayant une face postérieure (31) dont la forme est le négatif d'une zone d'une deuxième partie de l'os (H) du patient adjacente à ladite zone de la première partie de l'os (H), la deuxième partie (3) comprenant des orifices (32) pour la fixation au moyen de fils de fixation (A) à ladite deuxième partie de l'os (H) ;
la première partie (2) et la deuxième partie (3) étant configurées pour être couplées le long d'une rainure de coupe (4) servant de guide pour couper l'os (H) afin de séparer la première partie de l'os (H) de la deuxième partie de l'os (H), **caractérisé en ce que**
lorsque le guide chirurgical (1) est fixé à l'os (H) dans une position où la face postérieure (21) de la première partie (2) s'adapte sur la première partie de l'os (H) et la face postérieure (31) de la deuxième partie (3) s'adapte sur la deuxième partie de l'os (H), la première partie (2) et la deuxième partie (3) étant couplées l'une à l'autre, la rainure de coupe (4) est contenue dans un plan qui est incliné par rapport au plan sagittal et au plan coronal du patient ;
et **en ce que** la première partie (2) et la deuxième partie (3) comprennent des moyens configurés pour signaler une position où la deuxième partie de l'os (H) a pivoté d'un angle de rotation prédéterminé par rapport à la première partie de l'os (H) autour d'un axe qui est perpendiculaire audit plan incliné contenant la rainure de coupe (4).

2. Guide chirurgical (1) selon la revendication 1, dans lequel les moyens pour signaler le moment où la deuxième partie de l'os (H) a pivoté d'un angle de rotation prédéterminé par rapport à la première partie de l'os (H) comprennent :
- un premier orifice de référence (23) de la première partie (2) configuré pour l'insertion à travers celui-ci d'un premier fil de référence (A_{R1} ) dans la première partie de l'os (H) ; et
- un deuxième orifice de référence (33) de la deuxième partie (3) configuré pour l'insertion à travers celui-ci d'un deuxième fil de référence (A_{R2}) dans la deuxième partie de l'os (H),
de telle sorte que, une fois que la première partie (2) et la deuxième partie (3) du guide chirurgical (1) sont séparées de l'os (H) après que la coupe (C) contenue dans ledit plan incliné de la rainure de coupe (4) a été effectuée, une position relative particulière du deuxième fil de référence (A_{R2}) par rapport au premier fil de référence (A_{R1}) indique que la deuxième partie de l'os (H) a pivoté de l'angle de rotation prédéterminé par rapport à la première partie de l'os (H).

3. Guide chirurgical (1) selon la revendication 2, dans lequel la position relative du deuxième fil de référence (A_{R2}) par rapport au premier fil de référence (A_{R1}) indiquant que la deuxième partie de l'os (H) a pivoté de l'angle de rotation prédéterminé par rapport à la première partie de l'os (H) consiste en l'alignement, le long d'une direction parallèle à l'axe longitudinal (E_{L}) de l'os (H), du point d'insertion dans l'os (H) du deuxième fil de référence (A_{R2}) par rapport au point d'insertion dans l'os (H) du premier fil de référence (A_{R1}).

4. Guide chirurgical (1) selon l'une quelconque des revendications 2 à 3, dans lequel le premier orifice de référence (23) de la première partie (2) est situé dans une position adjacente à un bord inférieur (24) de la première partie (2) constituant une face supérieure de la rainure de coupe (4).

5. Guide chirurgical (1) selon l'une quelconque des revendications 2 à 4, dans lequel le deuxième orifice de référence (33) de la deuxième partie (3) est situé à une position adjacente à un bord supérieur (35) de ladite deuxième partie (3) constituant une face inférieure de la rainure de coupe (4).

6. Guide chirurgical (1) selon l'une quelconque des revendications 2 à 5, dans lequel une face antérieure (34) de la deuxième partie (3) comprend une marque (M) adjacente à un bord supérieur (35) de ladite deuxième partie (3) formant une face inférieure de la rainure de coupe (4), de telle sorte que ladite marque (M) indique la position où le premier fil de référence (A_{R1}) doit se trouver lorsque la deuxième partie de l'os (H) a pivoté de l'angle de rotation prédéterminé par rapport à la première partie de l'os (H).

7. Guide chirurgical (1) selon la revendication 6, dans lequel ladite marque (M) est une ligne droite reliant un point adjacent audit bord supérieur (35) de la deuxième partie (3) où le premier fil de référence (A_{R1}) doit être situé lorsque la deuxième partie de l'os (H) a été tournée de l'angle de rotation prédéterminé avec le deuxième orifice de référence (33) de la deuxième partie (3).

8. Guide chirurgical (1) selon l'une quelconque des revendications précédentes, dans lequel le bord supérieur (35) de la deuxième partie (3) comprend une saillie (38) configurée pour venir en butée contre le bord inférieur (24) de la première partie (2) afin de séparer lesdits bords (24, 35) lorsque les deux parties (2, 3) sont couplées pour former la rainure (4).

9. Guide chirurgical (1) selon la revendication 8, dans lequel le bord supérieur (35) de la première partie (3) comprend en outre un bossage (36) émergeant de la saillie (38) et le bord inférieur (24) de la première partie (2) comprend une cavité (26) ouverte vers la surface postérieure (21), dans lequel le bossage (36) et la cavité (26) sont complémentaires de telle sorte que, lorsque la deuxième partie (3) est couplée à la première partie (2), le bossage (36) pénètre dans la cavité (26) et glisse le long de ladite cavité (26) jusqu'à ce que la face antérieure (34) de la deuxième partie (3) vienne en butée contre une face postérieure (25) d'un corps auxiliaire (2b) de la première partie (2).

10. Guide chirurgical (1) selon l'une quelconque des revendications 8 à 9, dans lequel un bord postérieur de la saillie (38) est séparé de la face postérieure (31) de la deuxième partie (3) d'une distance prédéterminée de telle sorte que, lorsque la deuxième partie (3) est fixée sur la zone de la première partie de l'os (H) avec le bord supérieur (35) situé dans le plan de la coupe (C), la deuxième partie de l'os (H) heurte ledit bord arrière de la saillie (38) lorsqu'elle a été tournée de l'angle de rotation prédéterminé par rapport à la première partie de l'os (H).

11. Guide chirurgical (1) selon l'une quelconque des revendications précédentes, dans lequel la face antérieure (34) de la deuxième partie (3) comprend des broches de guidage (37) qui sont complémentaires des évidements (28) dans la face postérieure (25) du corps auxiliaire (2b) de la première partie (2) de telle sorte que, lorsque la deuxième partie (3) est couplée à la deuxième partie (2), les broches de guidage (37) s'emboîtent dans les évidements (28).
